(19) Europäisches Patentamt
European Patent Office
Office européen des brevets

(11) **EP 4 498 065 A1**

(12) **EUROPEAN PATENT APPLICATION**
published in accordance with Art. 153(4) EPC

(43) Date of publication:
**29.01.2025 Bulletin 2025/05**

(21) Application number: **23780873.8**

(22) Date of filing: **30.03.2023**

(51) International Patent Classification (IPC):
***G01N 21/17*** (2006.01)

(52) Cooperative Patent Classification (CPC):
**G01N 21/17**

(86) International application number:
**PCT/JP2023/013172**

(87) International publication number:
**WO 2023/190865 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 JP 2022055706**

(71) Applicant: **DAIKIN INDUSTRIES, LTD.
Osaka-shi, Osaka 530-0001 (JP)**

(72) Inventors:
• **NOUMI, Masao
Osaka-Shi, Osaka 530-0001 (JP)**
• **SAKAKURA, Atsushi
Osaka-Shi, Osaka 530-0001 (JP)**

(74) Representative: **Hoffmann Eitle
Patent- und Rechtsanwälte PartmbB
Arabellastraße 30
81925 München (DE)**

(54) **OPTICAL COHERENCE TOMOGRAPHY APPARATUS, OPTICAL COHERENCE TOMOGRAPHY SYSTEM, OPTICAL COHERENCE TOMOGRAPHY METHOD AND INSPECTION METHOD**

(57) The disclosure provides, for example, an optical coherence tomography device capable of performing tomographic imaging over a wide area at a time and of performing tomographic imaging of a distant object. The optical coherence tomography device includes an objective lens configured to focus light emitted from a light source onto a sample. The optical coherence tomography device is configured to perform tomographic imaging of the sample based on interference between sample light, which is reflected light from the sample, and reference light, which is reflected light from a reference surface provided between the objective lens and the sample. The sample light and the reference light each are to pass through the objective lens. The light emitted from the light source and passed through the objective lens is to illuminate the sample at a wide angle. The reference surface includes a light scatterer.

FIG.2

EP 4 498 065 A1

**Description**

TECHNICAL FIELD

The disclosure relates to optical coherence tomography devices, optical coherence tomography systems, optical coherence tomography methods, and inspection methods.

BACKGROUND ART

Optical coherence tomography (OCT) is mainly used for tomographic imaging of biological organs such as eyeballs in the medical field.
Known optical coherence tomography devices are configured, for example, to split the light from a light source by a device such as a beam splitter, to apply the resulting light beams separately to a sample and a reference mirror to obtain reflected light beams, and based on interference between these reflected light beams passed through respective optical paths, to perform tomographic imaging (for example, see Patent Literature 1).

CITATION LIST

- Patent Literature

Patent Literature 1: JP 2011-104127 A

SUMMARY OF INVENTION

- Technical Problem

The disclosure provides an optical coherence tomography device capable of performing tomographic imaging over a wide area at a time and of performing tomographic imaging of a distant object, as well as an optical coherence tomography system, optical coherence tomography method, and inspection method including or using the device.

- Solution to Problem

The disclosure relates to an optical coherence tomography device including an objective lens configured to focus light emitted from a light source onto a sample,

the optical coherence tomography device being configured to perform tomographic imaging of the sample based on interference between sample light, which is reflected light from the sample, and reference light, which is reflected light from a reference surface provided between the objective lens and the sample,
the sample light and the reference light each being to pass through the objective lens,
the light emitted from the light source and passed through the objective lens being to illuminate the sample at a wide angle,
the reference surface including a light scatterer.

The objective lens is preferably a short-focus lens or a wide-angle lens.
Also, the objective lens preferably has a variable focal point.
Also, the light scatterer preferably has a haze value of 5 to 95% in a wavelength range from 400 to 1750 nm.
Also, the light scatterer preferably has a total light transmittance of 10 to 90% in a wavelength range from 400 to 1750 nm.
Also, the reference surface is preferably a flat surface.
Also, the optical coherence tomography device preferably further includes a probe, wherein the objective lens is provided in the probe.
Also, the optical coherence tomography device includes a plurality of the probes.
Also, the plurality of the probes is preferably configured to provide sets of information based on respective light beams of different frequencies.
Also, the probe or the plurality of the probes is preferably provided in a mechanically movable manner.
The disclosure also relates to an optical coherence tomography system including the optical coherence tomography device and a moving mechanism configured to mechanically move the probe or the plurality of the probes of the optical coherence tomography device.

The disclosure also relates to an optical coherence tomography method including using the optical coherence tomography device or the optical coherence tomography system.

Tomographic imaging is preferably performed with the objective lens and the sample being apart at a distance of 2 cm or longer and shorter than 2 m.

Tomographic imaging is also preferably performed while the probe that includes the objective lens is mechanically moved.

Tomographic imaging is also preferably performed while the sample is mechanically moved.

Also preferably, a plurality of the probes each of which includes the objective lens is used to perform tomographic imaging with a plurality of light beams of different frequencies.

Also preferably, a plurality of the probes each of which includes the objective lens is used to perform tomographic imaging in a plurality of different directions.

The disclosure also relates to an inspection method including performing tomographic imaging of a sample by the optical coherence tomography method to acquire image data; and inspecting an internal condition of the sample based on the image data.

- Advantageous Effects of Invention

The disclosure can provide an optical coherence tomography device capable of performing tomographic imaging over a wide area at a time and of performing tomographic imaging of a distant object, as well as an optical coherence tomography system, optical coherence tomography method, and inspection method including or using the device.

BRIEF DESCRIPTION OF THE DRAWINGS

FIG. 1 is a schematic diagram of an example of a conventional optical coherence tomography (OCT) device.
FIG. 2 is a schematic diagram of an example of an OCT device of the disclosure.
FIG. 3 is a schematic diagram of another example of the OCT device of the disclosure.

DESCRIPTION OF EMBODIMENTS

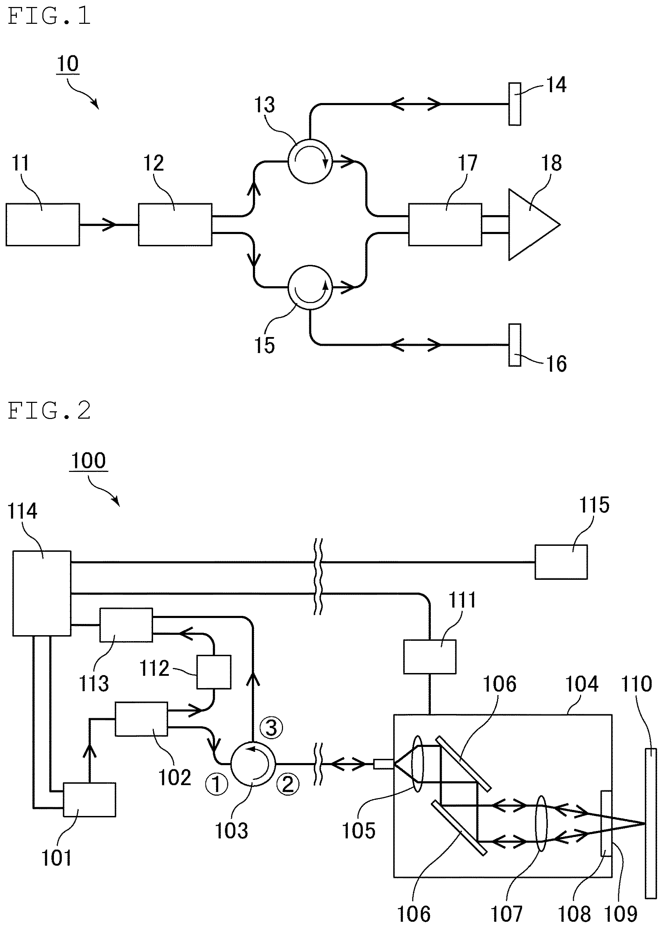

In the medical field, an optical coherence tomography (OCT) device including a Michelson interferometer as shown in FIG. 1 is commonly used. An OCT device 10 of FIG. 1 is configured such that the light emitted from a light source 11 is split by a coupler 12 into reference light, which is to pass through an optical path including a circulator 13 and a reference mirror 14, and sample light, which is to pass through an optical path including a circulator 15 and a sample 16. The reference light and the sample light are combined by a coupler 17 and an interference signal is detected by a photodetector 18.

OCT devices used in the medical field have a priority to perform close-range, high-precision tomographic imaging of a very small area, such as an eyeball, and are difficult to apply to fields involving tomographic imaging over a wide area at a time or tomographic imaging of a distant object.

The disclosers performed studies to find that the above issue can be solved by an OCT device that is configured such that both sample light and reference light are to pass through an objective lens, such that the light emitted from the light source and passed through the objective lens is to illuminate a sample at a wide angle, and such that a reference surface includes a light scatterer, thereby completing the OCT device of the disclosure.

Hereinafter, the disclosure is described in detail.

The disclosure provides an optical coherence tomography device including an objective lens configured to focus light emitted from a light source onto a sample, the OCT device being configured to perform tomographic imaging of the sample based on interference between sample light, which is reflected light from the sample, and reference light, which is reflected light from a reference surface provided between the objective lens and the sample, the sample light and the reference light each being to pass through the objective lens, the light emitted from the light source and passed through the objective lens being to illuminate the sample at a wide angle, the reference surface including a light scatterer.

The OCT device of the disclosure is configured such that the sample light, which is reflected light from a sample to be imaged, and the reference light, which is reflected light from a reference surface, both pass through the objective lens. This configuration can prevent difference in environmental conditions (e.g., temperature) between the sample light path and the reference light path even when a portion including the objective lens (e.g., a probe) is used at a place distant from the body (housing), resulting in a small shift in a tomographic image obtained.

The sample light and the reference light are incident on the objective lens through the surface close to the sample and are emitted therefrom through the surface close to the light source.

The sample light and the reference light are generated from the light emitted from the light source. The light emitted from the light source passes through the objective lens of the OCT device of the disclosure and focuses on the sample. The light reflected on the sample serves as the sample light. Part of the light emitted from the light source is reflected on a reference surface provided between the objective lens and the sample, serving as the reference light.

The sample light and the reference light are each preferably generated from the light emitted from the light source and passed through the objective lens. In comparison with conventional Michelson OCT devices in which the light is split before passing through the objective lens to separately generate the sample light and the reference light, the above configuration of the disclosure can reduce the difference in environmental conditions between the sample light path and the reference light path and can more greatly reduce a shift in a tomographic image obtained.

The OCT device of the disclosure is configured such that the light emitted from the light source and passed through the objective lens illuminates the sample at a wide angle. This configuration enables tomographic imaging over a wide area at a time as well as tomographic imaging of a distant object.

The phrase "illuminates the sample at a wide angle" herein means light is applied such that the light incident on the sample surface and the perpendicular line to the sample surface form an angle $\alpha$ within a range from 0 degrees or greater to 90 degrees or smaller. The angle $\alpha$ may be greater than 0 degrees, preferably 1 degree or greater, more preferably 2 degrees or greater, while it may be smaller than 90 degrees, preferably 30 degrees or smaller, more preferably 15 degrees or smaller.

Light may be applied to the entire of the aforementioned range.

Examples of methods of illuminating the sample at a wide angle include (i) a method in which the light emitted from the light source is converged with a short-focus lens, which serves as an objective lens, and the resulting converged light is reflected on a scanning mirror at a wide angle, so that the light illuminates the sample; and (ii) a method in which the light emitted from the light source is applied to the sample with a wide-angle lens, which serves as an objective lens.

Particularly preferred is the method (i). In the method (i), the objective lens (short-focus lens) can be provided in front of the scanning mirror, i.e., on the optical path between the light source and the scanning mirror. This can increase the flexibility in adjusting the angle of light applied to the sample and the distance to the sample.

In the method (i), light is applied while the tilt of the mirror surface of the scanning mirror is changed so that the angle $\alpha$ falls within the aforementioned range. Thereby, the light emitted from the light source can illuminate the sample at a wide angle.

In the method (ii), light is applied in a configuration such that the angle of view $\theta$ determined by the following method is 0 degrees or greater and 90 degrees or smaller. Thereby, the light emitted from the light source can illuminate the sample at a wide angle.

The angle of view $\theta$ may be greater than 0 degrees, preferably 1 degree or greater, more preferably 2 degrees or greater, while it may be smaller than 90 degrees, preferably 30 degrees or smaller, more preferably 15 degrees or smaller.

The angle of view $\theta$ is determined from the following $\tan\theta$ value with diagonally opposite ends of an image sensor of the OCT device being joined at the center of the lens.

$$\tan\theta = \text{(half of distance between opposite ends of image sensor)/(distance from center of lens to image sensor)}$$

The image sensor may be one provided on a detector (detector (1)) to be described later, and may be a CCD image sensor or an InGaAs photodiode.

Within the above wide-angle illumination range, preferably, the incident light flux sufficiently reaches the corners of the range that the objective lens can capture and the optical performance is ensured within this range. The shift in optical image formation, such as chromatic aberration or distortion, is preferably 10% or lower.

The objective lens is preferably a short-focus lens or a wide-angle lens.

The short-focus lens is a condenser lens having a focal length of 50 mm or shorter at infinity focus with light having a wavelength of 800 to 1750 nm. The focal length of the short-focus lens is more preferably 45 mm or shorter, while preferably 1 mm or greater, more preferably 2 mm or greater.

The focal length can be determined in accordance with JIS B 7094 (ISO 517).

The short-focus lens preferably has a variable focal point (condensing length). This variable focal point enables a change in the focal point without changing objective lenses.

Examples of the short-focus lens having a variable focal point include collimator lenses capable of adjusting the distance between the lens and the outlet of an optical fiber.

The short-focus lens also preferably has a variable focal length. This variable focal length enables a change in the focal length without changing objective lenses.

The short-focus lens may be a collimator lens such as an achromatic fiber collimator.

The short-focus lens can be particularly suitably used in the method (i).

The wide-angle lens may have a focal length of 200 mm or shorter at infinity focus with light having a wavelength of 800 to 1750 nm, preferably 100 mm or shorter, more preferably 50 mm or shorter, more preferably 40 mm or shorter. The focal length may be 1 mm or greater, preferably 2 mm or greater, more preferably 8 mm or greater.

The focal length can be determined in accordance with JIS B 7094 (ISO 517).

The wide-angle lens preferably has a variable focal point (focusing length). This variable focal point enables a change in the focal point without changing objective lenses.

The wide-angle lens also preferably has a variable focal length. This variable focal length enables a change in the focal length without changing objective lenses.

The wide-angle lens may be a wide-angle camera lens.

The wide-angle lens can be particularly suitably used in the method (ii).

The reference surface is provided between the objective lens and the sample. The reference surface may be provided perpendicularly to the optical axis of the objective lens, but is not limited to this configuration.

The reference surface is any surface that reflects at least part of the light emitted from the light source. In order to easily achieve a configuration in which the sample light and the reference light pass through a common optical path, the reference surface is preferably a surface that transmits part of the light emitted from the light source and reflects another part thereof. In this configuration, the light transmitted through the reference surface focuses on the sample to generate the sample light, while the light reflected on the reference surface serves as the reference light.

The reference surface is preferably a flat surface, more preferably a flat surface of a reference item, still more preferably the surface close to the sample of the reference item.

The reference item is preferably one that transmits part of the light emitted from the light source and reflects another part thereof.

The reference surface includes a light scatterer. The reference item may also include a light scatterer.

When the light emitted from the light source and passed through the objective lens illuminates the sample at a wide angle, this light emitted from the light source may be obliquely incident on the reference surface at some positions. Such oblique incidence of light on the reference surface may cause a difficulty in obtaining reflected light at a sufficient intensity along the same optical path as that of the incident light.

In the OCT device of the disclosure, the reference surface includes a light scatterer, which makes it easy to obtain, from the light obliquely incident on the reference surface, reflected light along the same optical path as that of the incident light. This configuration enables tomographic imaging over a wide area at a time as well as tomographic imaging of a distant object.

The light scatterer may have a haze value of 5 to 95% in a wavelength range from 400 to 1750 nm. The haze value is preferably 10% or higher, more preferably 15% or higher, while preferably 90% or lower, more preferably 80% or lower.

The haze value can be measured using a haze meter in conformity with JIS R 3106 (ISO 9050), JIS K 7361-1 (ISO 13468-1), and JIS K 7136 (ISO 14782).

The light scatterer may have a total light transmittance of 10 to 90% in a wavelength range from 400 to 1750 nm. The total light transmittance is preferably 15% or higher, more preferably 20% or higher, while preferably 80% or lower, more preferably 70% or lower.

The total light transmittance can be measured using a haze meter in conformity with JIS R 3106 (ISO 9050), JIS K 7361-1 (ISO 13468-1), and JIS K 7136 (ISO 14782).

The light scatterer may be any one that scatters the light emitted from the light source. Examples thereof include glass and resin having a surface with fine irregularities such as ground glass, frosted glass, and Fresnel lens; and glass and resin containing bubbles or particles that scatter light.

In particular, the light scatterer is preferably glass or resin having a surface with fine irregularities.

The reference item may have any shape including a flat surface, such as a plate shape, a cylindrical shape, or a prism shape, and preferably has a cylindrical shape. The cylindrical shape is not necessarily a perfectly circular cylindrical shape. In the case of a reference item including a different flat surface in addition to the reference surface, the reference surface and the different flat surface are not necessarily parallel to each other.

The reference item has a thickness (thickness in the optical axis direction) of preferably 0.01 to 50 mm. The thickness is more preferably 0.1 mm or greater, still more preferably 0.3 mm or greater, particularly preferably 0.5 mm or greater, while more preferably 30 mm or smaller, still more preferably 20 mm or smaller, particularly preferably 10 mm or smaller.

In the case where the reference item has a non-uniform thickness, the thickness at the thinnest portion and the thickness at the thickest portion are each preferably within the above range.

The reference item preferably satisfies the following relation (1):

$$nd \geq Z_{max} \quad (1)$$

wherein nd represents the optical thickness of the reference item; and $Z_{max}$ represents the measurable distance.

The optical thickness is the product of the refractive index and actual (geometric) thickness of the reference item.

The measurable distance is expressed by the following relation (2):

$$Z_{max} = c/(4\delta f) \qquad (2)$$

wherein c represents the speed of light; and δf represents the frequency interval of OCT interference signal sampling.

Using a reference item satisfying the relation (1) can prevent appearance of a signal based on back reflection on the back bottom surface (the surface opposite to the reference surface) of the reference item in a tomographic image (within the area corresponding to a depth of greater than 0 and smaller than $Z_{max}$), which can provide a tomographic image with a higher precision.

The reference item more preferably satisfies the following relation (3):

$$n \times WD > nd > n \times Z_{max} \qquad (3)$$

wherein n represents the refractive index of the reference item; WD represents the working distance of the OCT device; and nd and $Z_{max}$ are as defined above.

The working distance refers to the distance between the forefront surface close to the sample of the objective lens and the sample, with the sample being in focus.

Using a reference item satisfying the relation (3) can reduce the intensity of a ghost image based on back reflection on the back bottom surface (the surface opposite to the reference surface) of the reference item, which can provide a tomographic image with a much higher precision.

In order to more reduce the intensity of a ghost image based on back reflection, the thickness of the reference item is preferably as thick as possible within the range satisfying the relation (3). Also, preferably, the back bottom surface of the reference item is tilted relative to the reference surface.

The aforementioned effect is particularly significant in the case of placing an anti-aliasing filter (low-pass filter) to be described later.

The reference item particularly preferably satisfies the following relation (4):

$$nd = m \times Z_{max} \qquad (4)$$

wherein nd and $Z_{max}$ are as defined above; and m is an integer of 1 or greater.

In the formula, m is preferably an integer of 1 or greater and 20 or smaller, more preferably an integer of 1 or greater and 10 or smaller.

Using a reference item satisfying the relation (4) allows a signal based on back reflection on the back bottom surface (the surface opposite to the reference surface) of the reference item to overlap an edge of a tomographic image (the position corresponding to a depth of 0 or $Z_{max}$), which results in less impact on the tomographic image and can provide a tomographic image with a much higher precision.

The reference surface may be provided at a position apart from the objective lens by a distance of 5 mm to 5 m. The distance is preferably 10 mm or greater, more preferably 20 mm or greater, while preferably 2 m or smaller, more preferably 1 m or smaller.

The OCT device of the disclosure may include the reference surface (reference item).

The light source may be a low coherence light source, and is preferably a frequency-scanning light source configured to perform scanning while changing the frequency (wavelength) over time.

Examples of the frequency-scanning light source to be used include a wavelength-swept laser utilizing a wavelength-swept filter (e.g., driven with a polygonal mirror, driven with a galvanometer mirror), a FDML laser, a MEMS wavelength-swept light source (e.g., MEMS VCSEL, external cavity MEMS Fabry-Pérot laser), and an SGDBR laser.

Examples of the light beam emitted from the light source include visible light and infrared light. Near infrared light (NIR) is preferred. The light beam to be used is preferably a light beam having a wavelength of 800 to 2000 nm. In particular, from the viewpoint of stability of the light source and reliability of the sensor, more preferred is a light beam having a central wavelength of 940 ± 50 nm, 1100 ± 50 nm, 1310 ± 50 nm, 1550 ± 100 nm, or 1750 ± 100 nm.

The OCT device of the disclosure may include the light source.

The OCT device of the disclosure performs tomographic imaging of the sample based on the interference between the sample light and the reference light. The interference may be any one that allows both the sample light and the reference light to theoretically pass through the objective lens, preferably Fizeau interference or Mirau interference, more preferably Fizeau interference.

Examples of OCT types to be usable in the OCT device of the disclosure include time domain OCT (TD-OCT) and Fourier domain OCT (FD-OCT). Examples of the FD-OCT include spectral domain OCT (SD-OCT) and swept source OCT (SS-OCT). Owing to its high sensitivity and deep measurable depth, preferred among these is SS-OCT.

The OCT device of the disclosure preferably includes a probe, wherein the objective lens is provided in the probe. One probe may be provided or a plurality of probes may be provided.

The probe may or may not have the reference surface (or reference item), and preferably has the reference surface (or reference item). The probe more preferably further includes a collimator and a scanning mirror, each of which is to be described later.

In the case where the OCT device of the disclosure includes a plurality of the probes, this plurality of the probes enables simultaneous tomographic imaging at different positions.

Also, in the case where a plurality of the probes is provided, this plurality of the probes may be configured to provide sets of information based on respective light beams of different frequencies. This configuration enables simultaneous tomographic imaging at different depths with the plurality of the probes.

The probe or the plurality of the probes may be provided in a mechanically movable manner. This configuration enables tomographic imaging with the probe being mechanically moved, which makes it easy to perform tomographic imaging of a large sample.

The direction of moving the probe may be, but is not limited to, any direction determined in accordance with the area to be subjected to tomographic imaging. The direction may be, for example, a direction crossing the optical axis of the objective lens, a direction crossing the optical path of the light beam emitted from the objective lens toward the sample, a direction along the shape of the sample, a direction along the sample surface, or a direction substantially parallel with the sample surface.

This configuration can be achieved by providing the probe on a moving mechanism to be described later.

The probe is preferably coupled with the OCT device body (housing) via an optical fiber. In this configuration, the light emitted from the light source as well as the sample light and the reference light are transferred through the optical fiber.

In this configuration, an object to be imaged, which is placed far from the OCT device body, can be subjected to tomographic imaging by adjusting the length of the optical fiber so that the probe is placed near the object to be imaged. Since the light emitted from the light source as well as the sample light and the reference light are transferred through the optical fiber, no difference in environmental conditions occurs between the sample light path and the reference light path even when the optical fiber is long. This results in a small shift in a tomographic image obtained. Further, since the device is wired with the optical fiber, an object to be imaged placed far from the body can also be subjected to high-resolution OCT analysis.

The optical fiber may have any length that may be determined in accordance with the place of an object to be imaged. The length may be 1 m or longer, preferably 3 m or longer, more preferably 5 m or longer, still more preferably 10 m or longer, while it may be 100 m or shorter, or may be 50 m or shorter.

The OCT device body (housing) preferably includes the light source, for example, and more preferably includes components such as a circulator, a detector, a DAQ device, and an arithmetic logic unit to be described later.

The OCT device of the disclosure preferably further includes a collimator configured to convert the light emitted from the light source into parallel light. The collimator is preferably provided on the optical path between the light source and the reference surface, more preferably provided on the optical path between the light source and the objective lens.

The aforementioned objective lens (preferably short-focus lens) may be used as the collimator.

The OCT device of the disclosure preferably further includes a scanning mirror configured to scan the light emitted from the light source and focused on the sample. The scanning mirror is preferably provided on the optical path between the light source and the objective lens, and is more preferably provided on the optical path between the collimator and the objective lens. This configuration is particularly suitable for the case where the objective lens is a wide-angle lens.

The scanning mirror is alternatively preferably provided on the optical path between the objective lens and the reference surface. This configuration is particularly suitable for the case where the objective lens is a short-focus lens.

Examples of the scanning mirror include a galvanometer mirror, a polygonal mirror, and a MEMS mirror. Preferred among these is a galvanometer mirror, more preferred is a single-axis or two-axis galvanometer mirror, still more preferred is a two-axis galvanometer mirror.

The OCT device of the disclosure preferably further includes a driver for driving the scanning mirror.

The OCT device of the disclosure preferably further includes a circulator configured to emit the light emitted from the light source toward the objective lens and to emit the sample light and the reference light passed through the objective lens toward a detector configured to detect the sample light and the reference light. In this configuration, the sample light and the reference light can be transmitted with a single circulator. This configuration enables a smaller device and a lower cost than in the case of providing circulators separately for the sample light and the reference light as shown in FIG. 1.

The circulator preferably has three or more ports, more preferably three ports.

The circulator is preferably provided on the optical path between the light source and the objective lens, more preferably on the optical path between the light source and the collimator.

In the case of a three-port circulator, the light emitted from the light source is injected into a first port close to the light source and emitted from a second port close to the objective lens. The sample light and the reference light passed through the objective lens are injected into the second port and emitted from a third port close to the detector.

The OCT device of the disclosure preferably further includes a detector (also referred to as a detector (1)) configured to detect the sample light and the reference light. The detector (1) is preferably configured to detect an interference signal generated by the sample light and the reference light. One detector (1) may be provided or a plurality of detectors (1) may be provided.

The detector (1) is preferably a differential photodetector. The detector (1) may have a function to amplify a signal. Alternatively, an amplifier may be provided separately.

The OCT device of the disclosure preferably further includes a coupler (also referred to as a coupler (1)) configured to split the light emitted from the light source into split light 1 to be used for generating the sample light and the reference light and split light 2 to be used for removing a DC component of an interference signal. The coupler (1) is preferably provided on the optical path between the light source and the objective lens, more preferably provided on the optical path between the light source and the circulator.

The coupler (1), when provided, allows the split light 1 and the split light 2 to have an intensity ratio of preferably 90:10 to 99:1, more preferably 92:8 to 98:2. Light splitting with this intensity ratio enables effective removal of a DC component from an interference signal.

The OCT device of the disclosure preferably further includes a detector (also referred to as a detector (2)) configured to detect the split light 2. The detector (2) may be the same detector as the aforementioned detector (1) or may be a different detector.

The OCT device of the disclosure preferably further includes an attenuator configured to attenuate the split light 2. The attenuator is preferably a variable optical attenuator (VOA). The attenuator is preferably provided on the optical path between the coupler (1) and the detector (2) configured to detect the split light 2.

The OCT device of the disclosure may further include a polarizing module configured to adjust the polarization state of the light emitted from the light source. The polarizing module is preferably provided on the optical path between the light source and the objective lens, more preferably provided on the optical path between the light source and the coupler (1).

The OCT device of the disclosure, when provided with a plurality of the probes, preferably includes a coupler (also referred to as a coupler (2)) configured to split the light emitted from the light source into light components to be sent to the respective probes. The coupler (2) is preferably provided on the optical path between the light source and the objective lens, more preferably provided on the optical path between the circulator and the collimator.

The OCT device of the disclosure may include a modulator configured to modulate the frequency of light. Optical modulation with the modulator enables an unrestricted change of the depth shown as a tomographic image.

Examples of the modulator include an acousto-optic (AO) modulator and an electro-optic (EO) modulator.

In the case of providing a plurality of the probes, a plurality of the modulators may be provided correspondingly to the probes. This configuration enables acquisition of sets of information based on respective light beams of different frequencies from the plurality of the probes, resulting in simultaneous tomographic imaging at different depths with the plurality of the probes. The images corresponding to the different depths, which are obtained by the plurality of the probes, may be encoded in the depth direction, so that the resulting images may be simultaneously shown on a display to be described later.

The modulator is preferably provided on the optical path between the light source and the objective lens, more preferably provided on the optical path between the circulator and the collimator.

The OCT device of the disclosure, when provided with a plurality of the probes, preferably includes a coupler (also referred to as a coupler (3)) configured to combine the light components emitted from the plurality of the probes. Providing a coupler (3) can reduce the number of DAQ devices to be described later.

The aforementioned coupler (2) may also be used as a coupler (3).

The coupler (3) is preferably provided on the optical path between the light source and the objective lens, more preferably provided on the optical path between the circulator and the collimator.

The OCT device of the disclosure preferably further includes a data acquisition (DAQ) system configured to acquire an interference signal generated by the sample light and the reference light. The DAQ device preferably includes an A/D converter. The DAQ device is preferably configured to convert the acquired interference signal into digital data.

One DAQ device may be provided or a plurality of DAQ devices may be provided. In the case of providing a plurality of the probes, a plurality of the DAQ devices is preferably provided correspondingly to the probes so as to reduce the calculation load.

The OCT device of the disclosure preferably further includes an anti-aliasing filter (also referred to as a low-pass filter) configured to attenuate unnecessary frequency components exceeding the measurable distance ($Z_{max}$). The anti-aliasing filter is preferably provided on the optical path between the detector (1) and the DAQ device described above.

The OCT device of the disclosure preferably further includes an arithmetic logic unit configured to generate an optical coherence tomographic image based on an interference signal generated by the sample light and the reference light. The arithmetic logic unit images the interference signal according to the properties such as the intensity to generate an optical coherence tomographic image.

The OCT device of the disclosure preferably further includes a display configured to display an optical coherence tomographic image obtained. The display may be of a stationary type or a portable type. A portable one is preferred because the image can be checked at a site where the image is obtained. The connection with the arithmetic logic unit may be wired or wireless. One display may be provided or multiple displays may be provided.

FIG. 2 shows an example of the OCT device of the disclosure. The OCT device of the disclosure is not limited thereto.

An OCT device 100 in FIG. 2 is configured such that a frequency-scanning light source 101 emits light to be used for OCT. The frequency-scanning light source 101 outputs a trigger signal at every start of frequency scanning. A Mach-Zehnder interferometer detects the light and outputs a K-clock signal for equal frequency interval sampling.

The light emitted from the frequency-scanning light source 101 is split by a coupler 102 into split light 1 to be used for generating sample light and reference light and split light 2 to be used for removing a DC component of an interference signal at an intensity ratio of 95:5. The split light 1 is injected into a port 1 of a circulator 103 and emitted from a port 2, and is transferred to a probe 104 through an optical fiber having a length of several meters.

In the probe 104, the split light 1 is converted by a collimator 105 into parallel light, which is then reflected on a galvanometer mirror 106 and is incident on an objective lens 107, which is a wide-angle lens. The galvanometer mirror 106 is driven by a galvanometer mirror driver 111 and scans the parallel light in the X-Y direction, which is perpendicular to the optical axis. The parallel light incident on the objective lens 107 passes through a reference item 108, which includes a light scatterer, and focuses on a sample 110 to be imaged, and is then reflected on the surface of the sample and is incident on the objective lens 107 as sample light. Part of the parallel light incident on the objective lens 107 is reflected on a reference surface 109 of the reference item 108 and then incident on the objective lens 107 as reference light.

The sample light and the reference light incident on the objective lens 107 pass through the galvanometer mirror 106 and the collimator 105, and are then injected into the port 2 of the circulator 103 via the optical fiber, emitted from a port 3, and injected into a differential photodetector amplifier 113. The differential photodetector amplifier 113 detects and amplifies the interference signal based on the interference between the sample light and the reference light.

The split light 2 generated by the coupler 102 is attenuated by a variable optical attenuator 112 and then injected into the differential photodetector amplifier 113. The differential photodetector amplifier 113 utilizes the signal of the split light 2 to remove the DC component in the interference signal.

The amplified interference signal from which the DC component is removed by the differential photodetector amplifier 113 is collected and converted into digital data by a DAQ device (A/D converter) of a PC 114. Collection of the interference signal is initiated by a trigger signal emitted from the frequency-scanning light source 101 and is performed while synchronizing with the K-clock signal.

Between the differential photodetector amplifier 113 and the DAQ device is provided an anti-aliasing filter (not shown) that attenuates unnecessary frequency components exceeding the measurable distance ($Z_{max}$).

An arithmetic logic unit of the PC 114 generates an optical coherence tomographic image of the sample 110 based on the interference signal converted by the DAQ device and displays it on a mobile display 115.

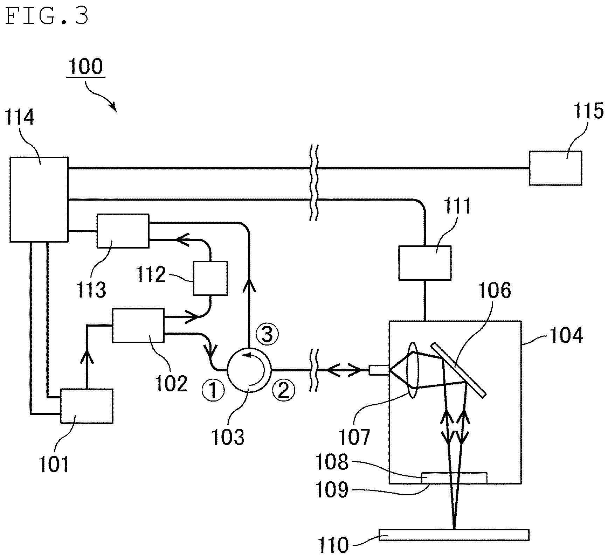

FIG. 3 shows another example of the OCT device of the disclosure. The OCT device of the disclosure is not limited thereto.

In the OCT device 100 of FIG. 3, light emitted from the objective lens 107, which is a short-focus lens, is reflected on the galvanic mirror 106, passes through the reference item 108, and then illuminates the sample 110. Changing the tilt of the galvanic mirror 106 with the galvanic mirror driver 111 enables applying light to the sample 110 at a wide angle.

The sample light from the sample surface is reflected on the galvanic mirror 106 and is incident on the objective lens 107. Part of the light emitted from the objective lens 107 is reflected on the reference surface 109 of the reference item 108 and then is incident on the objective lens 107 as reference light.

The other features are the same as described with reference to FIG. 2.

In the case where the OCT device of the disclosure is such that the probe and the OCT device body (housing) are coupled via an optical fiber having a length of 3 m or longer and that the difference in temperature is 1°C or greater between the atmosphere around the probe including the objective lens and the atmosphere around the OCT device body (housing), an optical coherence tomographic image obtained preferably has a shift of 100 um or smaller.

In the industrial field, for example, an object to be imaged may be placed far from the OCT device body, in the open air, or in a high- or low-temperature facility. In these cases, a great difference in environmental conditions (temperature) occurs between the probe and the OCT device body. Due to this difference in environmental conditions between the sample light path and the reference light path, an OCT device including the sample light path on the probe end and the reference light path on the body end provides a tomographic image with a great shift. In contrast, even in the above case, the OCT device of the disclosure causes no difference in environmental conditions between the sample light path and the reference light path, providing a tomographic image with a small shift.

In the above configuration, the length of the optical fiber is preferably 3 m or longer, more preferably 5 m or longer, still more preferably 10 m or longer, while it may be 100 m or shorter, or may be 50 m or shorter.

In the above configuration, the temperature difference between the atmospheres is preferably 1°C or greater, more preferably 5°C or greater, still more preferably 10°C or greater. The temperature difference is also preferably 50°C or smaller.

The shift of the optical coherence tomographic image is preferably 100 um or smaller, more preferably 50 um or smaller, particularly preferably 30 um or smaller.

The shift (ΔZ) is defined by the following equation (A):

$$\Delta Z \; (\mu m) \; = \; dn/dT \; (1/°C) \; \times \; L \; (m) \; \times \; 10^6 \; \times \; 2 \; \times \; \Delta t \; (°C) \qquad (A)$$

wherein dn/dT represents the temperature coefficient (1/°C) of the refractive index of the optical fiber material; L represents the length (m) of the optical fiber; and Δt represents the temperature difference (°C) between the sample light path and the reference light path.

ΔZ refers to the shift in the optical distance.

In the case where the optical fiber material is quartz glass, the optical wavelength is 1.3 um, and the temperature is around room temperature, dn/dT is about $1.9 \times 10^{-5}$ (1/°C).

In the case of an OCT device including the sample light path on the probe end and the reference light path on the body end, the temperature difference between the atmosphere around the probe and the atmosphere around the OCT device body is almost directly reflected in the temperature difference Δt between the optical paths. This causes a great shift ΔZ in a tomographic image obtained. In contrast, in the case of the OCT device of the disclosure, the temperature difference Δt between the optical paths is significantly small even when the temperature difference is large between the atmosphere around the probe and the atmosphere around the OCT device body. This results in a significantly small shift ΔZ.

The OCT device of the disclosure is preferably configured such that the area in the plane direction to be imaged at an optical resolution of 10 um or longer per tomographic imaging session under the following conditions is 0.1 to 100 cm in length and 0.1 to 100 cm in width. This enables high-precision tomographic imaging over a wide area at a time (even with a different light source).

(Light source)

High speed swept source available from Axsun Technologies (central wavelength: 1310 nm, sweep width: 100 nm, A-scan rate: 50 kHz, output: 25 mW, coherence length: 12 mm)

(Distance between objective lens and sample)

200 cm

The disclosure also provides an OCT system including the aforementioned OCT device of the disclosure and a moving mechanism configured to mechanically move the probe of the OCT device.

This OCT system of the disclosure enables tomographic imaging with the probe being mechanically moved, which makes it easy to perform tomographic imaging of a large sample.

The direction of moving the probe may be, but is not limited to, any direction determined in accordance with the area to be subjected to tomographic imaging. The direction may be, for example, a direction crossing the optical axis of the objective lens, a direction crossing the optical path of the light beam emitted from the objective lens toward the sample, a direction along the shape of the sample, a direction along the sample surface, or a direction substantially parallel with the sample surface.

The moving mechanism may be a mechanism configured to automatically move the probe.

The OCT device or OCT system of the disclosure can be used to perform optical coherence tomographic imaging of a sample. The disclosure also provides an OCT method using the aforementioned OCT device or OCT system of the disclosure.

The OCT method of the disclosure enables tomographic imaging over a wide area at a time and tomographic imaging of a distant object.

In the case of performing tomographic imaging at a place distant from the OCT device body, no difference in environmental conditions occurs between the sample light path and the reference light path, providing a tomographic image with a small shift.

The OCT method of the disclosure enables tomographic imaging with the objective lens and the sample being apart from each other by a distance of 2 cm or longer and shorter than 2 m. The distance between the objective lens and the sample may be greater than 3 cm, or may be 5 cm or greater, or may be 10 cm or greater, or may be 20 cm or greater.

In the OCT method of the disclosure, the OCT device or OCT system of the disclosure is used. This enables tomographic imaging of a sample at a distant place as described above.

The OCT method of the disclosure enables tomographic imaging with the reference surface and the sample being apart from each other by a distance of 0 to 50 mm. The distance between the reference surface and the sample is preferably 0.01 mm or greater, more preferably 0.1 mm or greater, while preferably 30 mm or smaller, more preferably 10 mm or smaller.

In the OCT method of the disclosure, tomographic imaging may be performed while the probe including the objective lens is mechanically moved. This configuration enables tomographic imaging with the probe being mechanically moved, which makes it easy to perform tomographic imaging of a large sample.

The direction of moving the probe may be, but is not limited to, any direction determined in accordance with the area to be subjected to tomographic imaging. The direction may be, for example, a direction crossing the optical axis of the objective lens, a direction crossing the optical path of the light beam emitted from the objective lens toward the sample, a direction along the shape of the sample, a direction along the sample surface, or a direction substantially parallel with the sample surface.

In this configuration, the probe may be moved while provided on the aforementioned moving mechanism.

In the OCT method of the disclosure, tomographic imaging may be performed while the sample is mechanically moved. This configuration enables tomographic imaging of a sample (e.g., product) that is being mechanically moved in a production line, for example.

The direction of moving the sample may be, but is not limited to, any direction determined in accordance with the area to be subjected to tomographic imaging. The direction may be, for example, a direction crossing the optical axis of the objective lens, a direction crossing the optical path of the light beam emitted from the objective lens toward the sample, a direction along the shape of the sample, a direction along the sample surface, or a direction substantially parallel with the sample surface.

In this configuration, the probe may be fixed or may be moved during tomographic imaging.

In the OCT method of the disclosure, a plurality of the probes each of which includes the objective lens may be used to perform tomographic imaging with a plurality of light beams of different frequencies. This configuration enables simultaneous tomographic imaging at different depths with the plurality of the probes.

In this configuration, the plurality of the probes may provide sets of information based on respective light beams of different frequencies and these sets of information may be used to perform tomographic imaging.

In the OCT method of the disclosure, a plurality of the probes each of which includes the objective lens may be used to perform tomographic imaging in a plurality of different directions. This configuration enables simultaneous tomographic imaging of a single sample in different directions with the plurality of the probes.

The disclosure also provides an inspection method including performing tomographic imaging of a sample by the aforementioned OCT method of the disclosure to acquire image data; and inspecting an internal condition of the sample based on the image data.

The inspection method of the disclosure enables inspection of an internal condition of a sample over a wide area at a time and inspection of an internal condition of a distant sample.

In the case of inspection at a place distant from the OCT device body, no difference in environmental conditions occurs between the sample light path and the reference light path, providing a tomographic image with a small shift and high-precision inspection.

The inspection may be inspection of a defect inside the sample. Examples of the defect include foreign substances and voids.

The OCT device, OCT system, OCT method, and inspection method of the disclosure can be suitably applied to the whole range of fields where the OCT is applicable. As described above, the disclosure enables tomographic imaging over a wide area at a time and tomographic imaging of a distant object. Also, the disclosure provides a tomographic image with a small shift even in the case of tomographic imaging at a place distant from the OCT device body. The disclosure is therefore suitably used particularly in the industrial field. The OCT device, OCT system, OCT method, or inspection method of the disclosure may be installed in the production line of an industrial product.

It should be appreciated that a variety of modifications and changes in the configurations and other details may be made to the aforementioned embodiments without departing from the spirit and scope of the claims.

REFERENCE SIGNS LIST

10: OCT device
11: light source
12, 17: coupler
13, 15: circulator
14: reference mirror

16: sample
18: photodetector
100: OCT device
101: frequency-scanning light source
102: coupler
103: circulator
104: probe
105: collimator
106: galvanometer mirror
107: objective lens
108: reference item
109: reference surface
110: sample
111: galvanometer mirror driver
112: variable optical attenuator
113: differential photodetector amplifier
114: PC
115: mobile display

**Claims**

1.  An optical coherence tomography device comprising an objective lens configured to focus light emitted from a light source onto a sample,

    the optical coherence tomography device being configured to perform tomographic imaging of the sample based on interference between sample light, which is reflected light from the sample, and reference light, which is reflected light from a reference surface provided between the objective lens and the sample,
    the sample light and the reference light each being to pass through the objective lens,
    the light emitted from the light source and passed through the objective lens being to illuminate the sample at a wide angle,
    the reference surface including a light scatterer.

2.  The optical coherence tomography device according to claim 1,
    wherein the objective lens is a short-focus lens or a wide-angle lens.

3.  The optical coherence tomography device according to claim 1 or 2,
    wherein the objective lens has a variable focal point.

4.  The optical coherence tomography device according to any one of claims 1 to 3,
    wherein the light scatterer has a haze value of 5 to 95% in a wavelength range from 400 to 1750 nm.

5.  The optical coherence tomography device according to any one of claims 1 to 4,
    wherein the light scatterer has a total light transmittance of 10 to 90% in a wavelength range from 400 to 1750 nm.

6.  The optical coherence tomography device according to any one of claims 1 to 5,
    wherein the reference surface is a flat surface.

7.  The optical coherence tomography device according to any one of claims 1 to 6, further comprising a probe, wherein the objective lens is provided in the probe.

8.  The optical coherence tomography device according to claim 7,
    wherein the optical coherence tomography device includes a plurality of the probes.

9.  The optical coherence tomography device according to claim 8,
    wherein the plurality of the probes is configured to provide sets of information based on respective light beams of different frequencies.

10. The optical coherence tomography device according to any one of claims 7 to 9,
   wherein the probe or the plurality of the probes is provided in a mechanically movable manner.

11. An optical coherence tomography system comprising:

   the optical coherence tomography device according to claim 10; and
   a moving mechanism configured to mechanically move the probe or the plurality of the probes of the optical coherence tomography device.

12. An optical coherence tomography method comprising using the optical coherence tomography device according to any one of claims 1 to 10 or the optical coherence tomography system according to claim 11.

13. The optical coherence tomography method according to claim 12,
   wherein tomographic imaging is performed with the objective lens and the sample being apart at a distance of 2 cm or longer and shorter than 2 m.

14. The optical coherence tomography method according to claim 12 or 13,
   wherein tomographic imaging is performed while the probe that includes the objective lens is mechanically moved.

15. The optical coherence tomography method according to claim 12 or 13,
   wherein tomographic imaging is performed while the sample is mechanically moved.

16. The optical coherence tomography method according to any one of claims 12 to 15,
   wherein a plurality of the probes each of which includes the objective lens is used to perform tomographic imaging with a plurality of light beams of different frequencies.

17. The optical coherence tomography method according to any one of claims 12 to 16,
   wherein a plurality of the probes each of which includes the objective lens is used to perform tomographic imaging in a plurality of different directions.

18. An inspection method comprising:

   performing tomographic imaging of a sample by the optical coherence tomography method according to any one of claims 12 to 17 to acquire image data; and
   inspecting an internal condition of the sample based on the image data.

FIG.1

FIG.2

FIG.3

| INTERNATIONAL SEARCH REPORT | International application No. |
|---|---|
| | **PCT/JP2023/013172** |

**A. CLASSIFICATION OF SUBJECT MATTER**

*G01N 21/17*(2006.01)i
FI:   G01N21/17 630

According to International Patent Classification (IPC) or to both national classification and IPC

**B. FIELDS SEARCHED**

Minimum documentation searched (classification system followed by classification symbols)

G01N21/17; G01N21/45; A61B1/00; A61B3/10; A61B10/00; G01B9/02; G01J3/45; G01J9/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2004-191114 A (TANNO, Naohiro) 08 July 2004 (2004-07-08)<br>paragraphs [0030]-[0035], fig. 2 | 1-18 |
| A | JP 2015-92158 A (TOMEY CORP) 14 May 2015 (2015-05-14)<br>paragraphs [0173]-[0177], fig. 14 | 1-18 |
| A | US 2004/0075843 A1 (MARRON, JOSEPH C.) 22 April 2004 (2004-04-22)<br>paragraphs [0014], [0017], [0028]-[0036], fig. 3 | 1-18 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **31 May 2023** | **13 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/013172**

| Patent document cited in search report | | | Publication date (day/month/year) | Patent family member(s) | | | Publication date (day/month/year) |
|---|---|---|---|---|---|---|---|
| JP | 2004-191114 | A | 08 July 2004 | (Family: none) | | | |
| JP | 2015-92158 | A | 14 May 2015 | US | 2015/0124261 | A1 | |
| | | | | paragraphs [0179]-[0183], fig.14 | | | |
| | | | | US | 2016/0045106 | A1 | |
| | | | | US | 2016/0161244 | A1 | |
| | | | | EP | 2869020 | A1 | |
| | | | | EP | 3205976 | A1 | |
| | | | | JP | 2017-173305 | A | |
| | | | | JP | 2019-168461 | A | |
| | | | | JP | 2019-215375 | A | |
| | | | | JP | 2020-24222 | A | |
| | | | | JP | 2021-56248 | A | |
| US | 2004/0075843 | A1 | 22 April 2004 | WO | 2004/003463 | A2 | |

Form PCT/ISA/210 (patent family annex) (January 2015)

**EP 4 498 065 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011104127 A